# EUROPEAN PATENT SPECIFICATION

(11) **EP 3 612 231 B1**
(45) Date of publication and mention of the grant of the patent: **25.06.2025**
(21) Application number: 18720377.3
(22) Date of filing: 20.04.2018
(51) Int. Cl.: A61K 47/46, A61K 9/50, A61P 35/00

(54) **DRUG DELIVERY SYSTEM**
WIRKSTOFFFREISETZUNGSSYSTEM
SYSTÈME D'ADMINISTRATION DE MÉDICAMENT

(30) Priority: 20.04.2017 IT 201700043316
(43) Date of publication of application: 26.02.2020
(73) Proprietor: Lipogems International S.p.A., 20129 Milano (IT)
(72) Inventor: PESSINA, Augusto, 20900 Monza (IT); ALESSANDRI, Giulio, 20131 Milano (IT); TREMOLADA, Carlo Ferdinando Maria, 20129 Milano (IT); ZEIRA, Offer, 20078 San Colombano al Lambro (Milano) (IT)
(74) Representative: Pace Napoleone, Maria
(86) International application number: PCT/IB2018/052751
(87) International publication number: WO 2018/193413

(56) References cited:
- WO-A1-2014/064642
- WO-A2-2011/154505
- CARLO TREMOLADA ET AL: "Adipose Tissue and Mesenchymal Stem Cells: State of the Art and Lipogems Technology Development", CURRENT STEM CELL REPORTS, vol. 2, no. 3, 13 July 2016 (2016-07-13), pages 304 - 312, XP055431510, DOI: 10.1007/s40778-016-0053-5

## Description

The present invention refers to a delivery system for molecules, preferably drugs, said system comprising fat tissue or derivatives thereof.

Moreover, the present invention refers to a fat-based delivery system, preferably loaded with molecules having antitumor activities, for use in the treatment of cancers.

### BACKGROUND

Drugs have long been used to improve health and extend lives.

It is well known that the delivering approach can influence significantly the efficacy of a drug. Indeed, some drugs are characterized by an optimum concentration range within which maximum benefit is derived, and concentrations out this range can be toxic or produce no therapeutic benefit at all.

On the other hand, the very slow progress in the efficacy of the treatment of diseases has increased the need for a multidisciplinary approach to the delivery of therapeutics to targets in tissues.

This need has caused a dramatic change in the practice of drug delivery in the past few decades and even greater changes are anticipated in the near future.

In particular, a distinct concept and delivery method has been recently developed that is based on the use of cells as therapeutic carriers. Cell-based vehicles are particularly attractive for delivery of bio-therapeutic agents that are difficult to synthesize, have reduced half-lives, limited tissue penetrance or are rapidly inactivated upon direct in vivo introduction.

The use of a physiological carrier to deliver therapeutics throughout the body to both improve their efficacy while minimizing inevitable adverse side effects, is an appealing perspective that can be applied in many clinical settings.

WO 2011/154505 A relates to a product comprising a cellular carrier including physiological mesenchymal cells, and a cytoxic chemotherapeutic drug internalized by said physiological mesenchymal cells.

The present invention proposes as solution to the need reported above a delivery system made of fat tissue or derivatives thereof. In particular, the fat tissue is micro-fragmented fat tissue and/or micro-fragmented lipoaspirate.

Indeed, the authors of the present invention have surprisingly found that:
1) A delivering system (scaffold) of isolated fat tissue, preferably micro-fragmented fat tissue, is rapidly loaded with molecules/drugs (the loading/priming phase takes minutes/hours);
2) The loaded molecules/drugs are released in a therapeutically effective dose and in long-lasting manner;
3) The loaded molecules/drugs keep their biological activity after release;
4) The decellularization (disruption of the cellular components) of the fat tissue, preferably the micro-fragmented fat tissue, does not affect the release of the molecules/drugs meaning that, advantageously, the scaffold, as disclosed in this context, eventually loaded with molecules/drugs, can be also kept frozen and used at later stages;
5) Such a kind of delivery system can be loaded with a broad spectrum of molecules/drugs;
6) The delivery system of the invention and the products of its degradation are biocompatible and show irrelevant toxic effects; and
7) The delivery system of the invention is a natural part of the body and therefore biodegradable besides no immunogenic.

In particular, the effects reported above, have been experimentally demonstrated by using both lipoaspirate as such and micro-fragmented lipoaspirate, that is the fat tissue obtained after the controlled micro-fragmentation process of the lipoaspirate performed as disclosed below.

However, the micro-fragmented fat delivery system is more advantageous than lipoaspirate (fat tissue) as such since it is easier to be handled and it allows a better standardization and reliability of the therapeutic results. In this regard, it is well known how important the standardization and reliability for any therapeutic use are.

### SUMMARY OF THE INVENTION

A first aspect of the present invention refers to a scaffold loaded with drugs, wherein said scaffold is isolated fat tissue, wherein the isolated fat tissue is micro-fragmented fat or a micro-fragmented lipoaspirate and/or clusters of micro-fragmented fat/lipoaspirate comprising Mesenchymal Stem Cells (MSCs), Adipose-derived Stem Cells (ASCs), Adipose Stem Cells, pericytes, adipocytes and endothelial cells and wherein said drugs are selected from: antiinflammatory molecules, antibiotics, anti-cancer molecules, and 5a-Reductase inhibitors.

The tissue is micro-fragmented fat or micro-fragmented lipoaspirate, preferably isolated from any animal, more preferably it is isolated from humans said humans being alive or cadaver and/or preferably comprising clusters of micro-fragmented fat/lipoaspirate having size ranging preferably from 10 to 5000 µm, more preferably from 100 to 3000 µm, still more preferably from 200 to 2500 µm, more preferably from 300 to 1500 µm, more preferably from 400 to 900 µm.

The anti-cancer molecules (chemotherapeutics) are preferably selected from: natural products, preferably vinca alkaloids, more preferably selected from: vinblastine, vincristine, and vinorelbine, taxane, preferably paclitaxel or docetaxel, vincristin, vinblastin, nocodazole, epothilones and navelbine, epidipodophyllotoxins (teniposide), actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamineoxaliplatin, iphosphamide, melphalan, merchlorethamine, mitomycin, mitoxantrone, nitrosourea, plicamycin, procarbazine, teniposide, triethylenethiophosphoramide and etoposide (VP16)), adriamycin, amsacrine, camptothecin, daunorubicin, dactinomycin, doxorubicin, eniposide, epirubicin, etoposide, idarubicin, irinotecan (CPT-11) and mitoxantrone, pemetrexed, 5-FU, rafenib, metotrexate, cyclophosphamide, bortezomib, tomozolomide, sorafenib. and any combination thereof.

More preferably, the anti-cancer molecules are selected from: Paclitaxel (PTX - Taxol or Onxal) or derivatives thereof, preferably Abraxane, Docetaxel and/or doxorubicin or derivative thereof, preferably Adriamycin, Vincristine.

According to a preferred embodiment, the amount of said molecules/drugs ranges from 1 to 5 mg/ml, preferably the amount of Paclitaxel (PTX - Taxol or Onxal) and derivatives thereof, preferably Abraxane, Docetaxel, for obtaining an anti-cancer effect/activity is not less than 150 ng for 100µl of said micro-fragmented fat tissue or micro-fragmented lipoaspirate and/or not less than 300 ng for 100µl of fat tissue or lipoaspirate sample.

According to a preferred embodiment, the amount of said molecules/drugs released per day ranges from 10-15 % compared to the loading/priming amount molecules/drugs.

A further aspect of the present invention, refers to the scaffold loaded with anti-cancer molecules/or drugs as disclosed before, for use in the treatment of a cancer, wherein said cancer is preferably selected from: renal cell cancer, Kaposi's sarcoma, chronic leukemia, prostate cancer, breast cancer, sarcoma, pancreatic cancer, ovarian carcinoma, rectal cancer, throat cancer, melanoma, colon cancer, bladder cancer, mastocytoma, lung cancer, mammary adenocarcinoma, pancreatic adenocarcinoma, myeloma, lymphoma, pharyngeal squamous cell carcinoma, and gastrointestinal or stomach cancer, more preferably selected from: pancreatic cancer, glioblastoma, neuroblastoma and mesotelioma.

### DRAWINGS

The invention will be disclosed in more detail by means of non-limiting examples referring to the following figures.

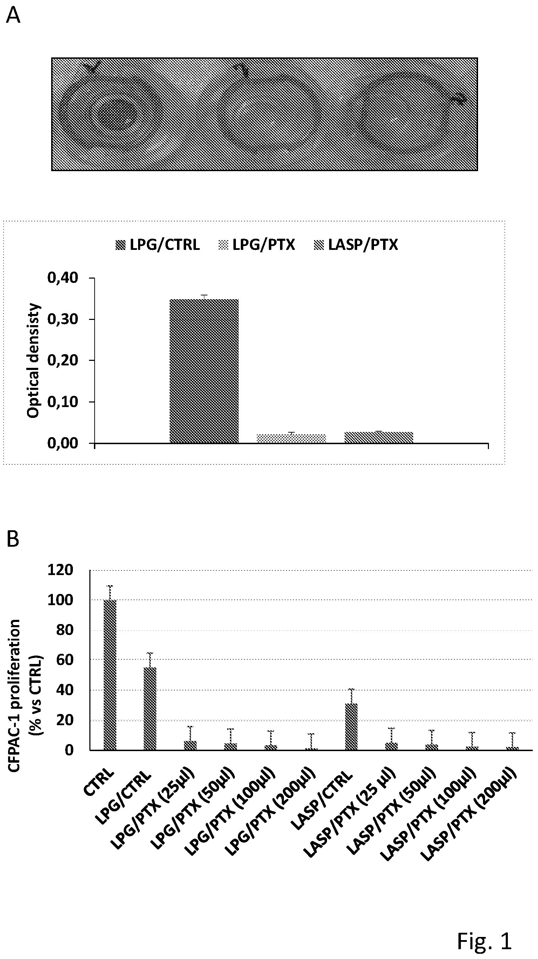

- Figure 1 shows the *in vitro* inhibition of cancer cell growth, in particular, the growth of pancreatic cancer cells (CFPAC1), by increasing amount of LPG/PTX and LASP/PTX. Tumor cell growth was evaluated by crystal violet (CV) staining. Fig. 1B shows the optical density of the CV eluted with acetic acid and measured at 550 nm. Fig. 1C shows the anticancer activity tested with MTT assay. The results show that increasing amount (25, 50, 100 and 200 µl) of LPG/PTX or LASP/PTX induce cell growth inhibition.

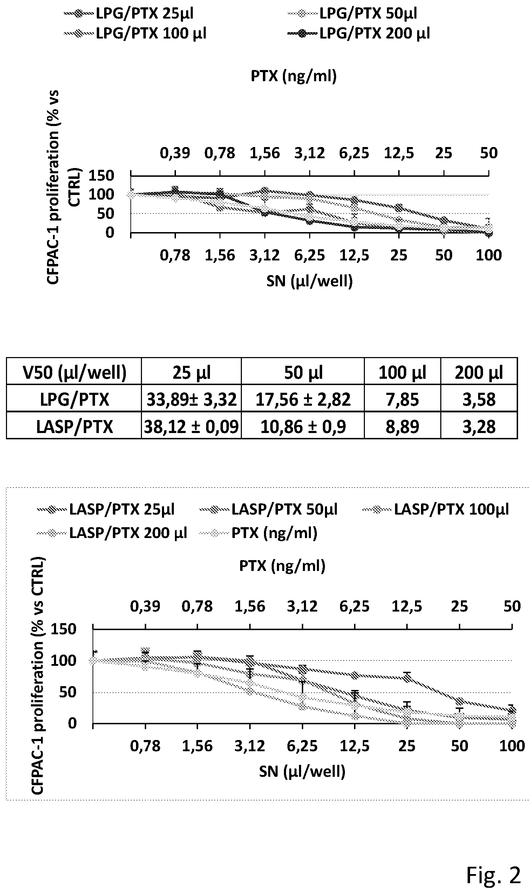

- Figure 2 shows the biological dosage of PTX released in the medium by different amount of LPG/PTX (A) and LASP/PTX (B)( from 0.78 to 100 µl). The dosage has been evaluated by using MTT assay measuring the PTX activity against CFPAC1 cells. Table in C reports the V50 values (volume inhibiting 50% cell growth) of different amount of LPG/PTX and LASP/PTX.

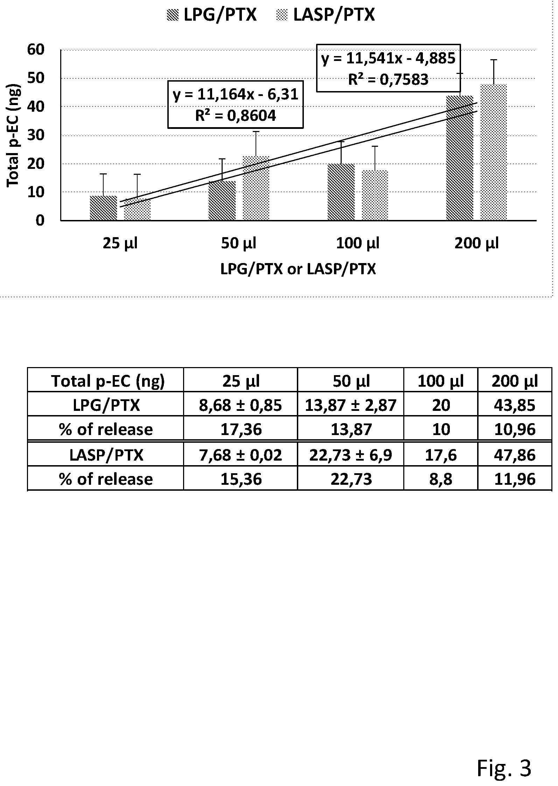

- Figure 3 shows the PTX releasing kinetics of different amount of LPG/PTX and LASP/PTX. The histogram (A) reports the total Paclitaxel equivalent concentration (p-EC) values of LPG/PTX and LASP/PTX.
   The R2 value means the correlation coefficient of the dose-response kinetics. Table (B) reports the single p-EC values and the release of PTX as percentage referred to the control (CTRL - 100%).

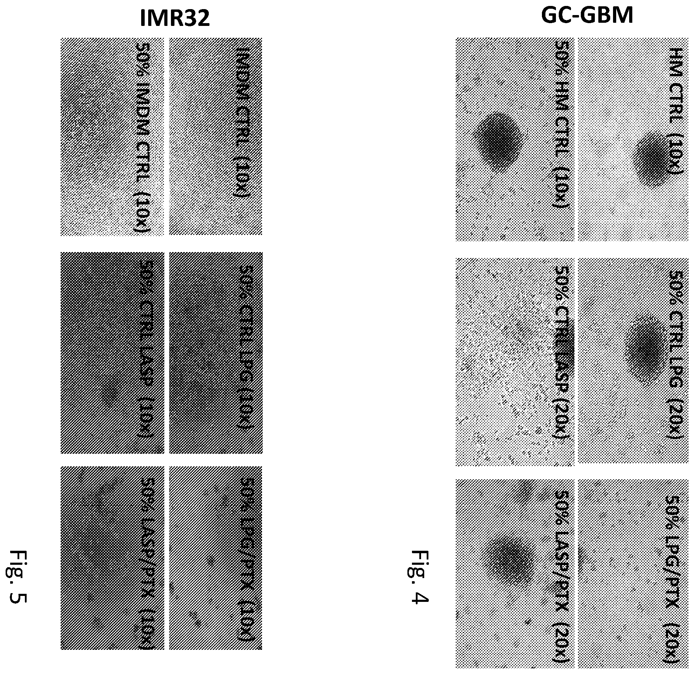

- Figure 4 shows that LPG/PTX and LASP/PTX inhibit the growth of primary GBM (GC-GBM) cancer cells. The photos refer to GC-GBM cells treated or not with 50% dilution of SN, derived from control and LPG/PTX or LASP/PTX (loaded with 2ug/ml). The results show the complete death of GC-GBM cells following LPG/PTX and LASP/PTX addition. SN derived from control fat tissue does not affect GC-GBM cell growth. No difference compared control medium (CTRL). Photos were acquired at 72h after treatments.
- Figure 5 shows that LPG/PTX and LASP/PTX induce IMR32 growth inhibition. Photos refer to IMR32 grown with or without 50% dilution of SN derived from LPG/PTX or LASP/PTX (loaded with 2µg/ml) and control culture. The results show that both LPG/PTX and LASP/PTX produced a IMR32 cell death. The control CM derived from not loaded fat tissue did not affect IMR32 cell growth. Photos were acquired at 72h after treatments.

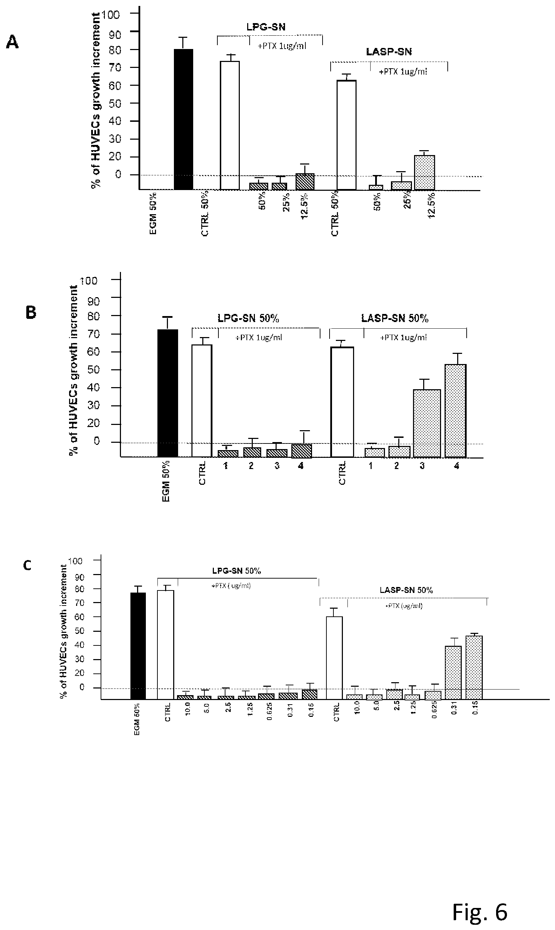

- Figure 6 shows that both LPG/PTX and LASP/PTX release the drug in a dose dependent manner and they induce long term IMR32 growth inhibition besides to inhibit angiogenesis. Fig. 6A shows a dose dependent activity of LPG/PTX and LASP/PTX. The results show that the priming of 100ul LPG with 300ng of PTX is sufficient to block IMR32 growth. Fig.6B shows the long lasting (even after four weeks) anti-tumor activity of LPG/PTX and LASP/PTX. Fig 6C shows that the SN derived from both LPG/PTX and LASP/PTX is able to inhibit endothelial cells (HUVECs) proliferation.

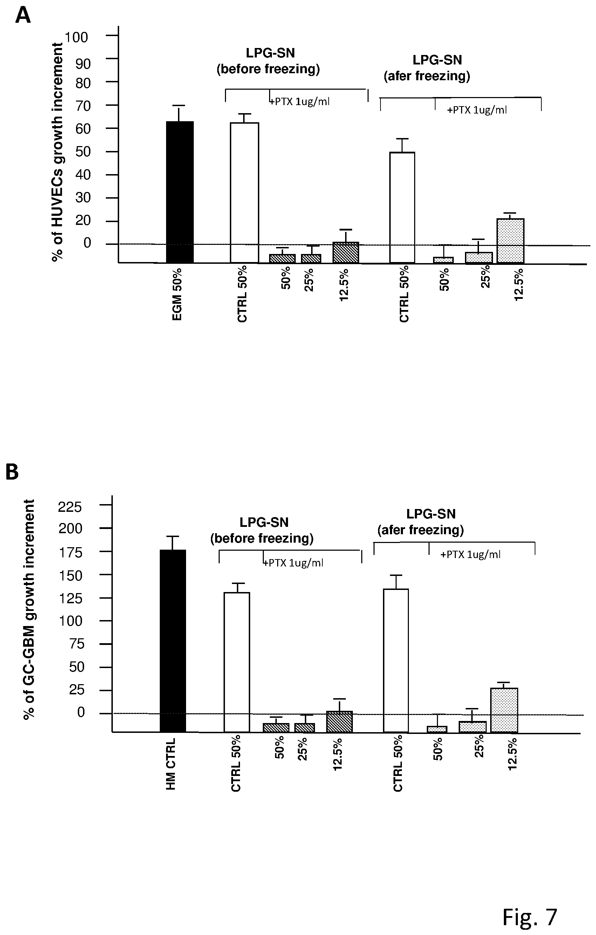

- Figure 7 shows that LPG/PTX freezing did not affect its anti-angiogenic and anti-tumor activity. After priming with PTX, LPG was maintained for 2 weeks at -20° C, then thawed and tested. Fig 7A shows the anti-angiogenic activity of SN derived from LPG/PTX (tested at different dilutions on HUVECs) before and after freezing. Fig. 7B shows the anti-tumor activity tested on GC-GBM cells.

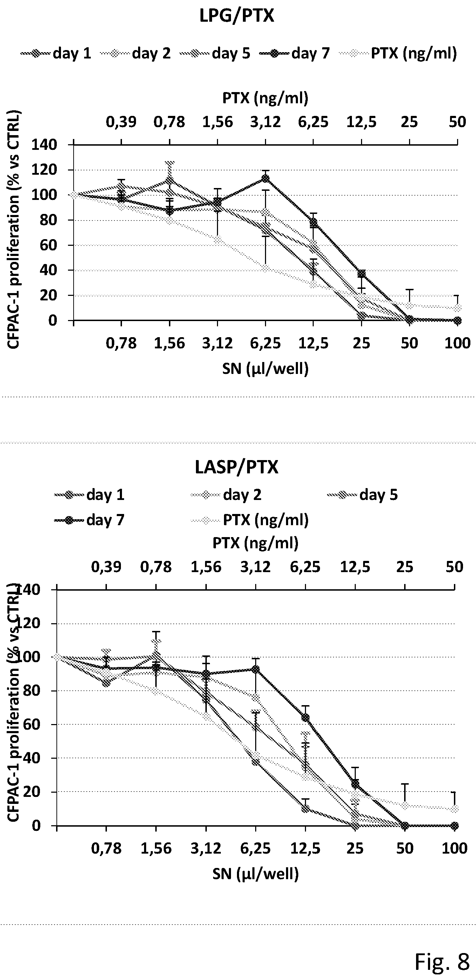

- Figure 8 shows the time releasing kinetics of PTX from both LPG/PTX and LASP/PTX. The anticancer activity on CFPAC1 cells has been assessed by MTT assay. The test measures the drug released in SN (supernatant) from both LPG/PTX (A) and LASP/PTX (B) at day 1, 2, 5 and 7 of incubation.

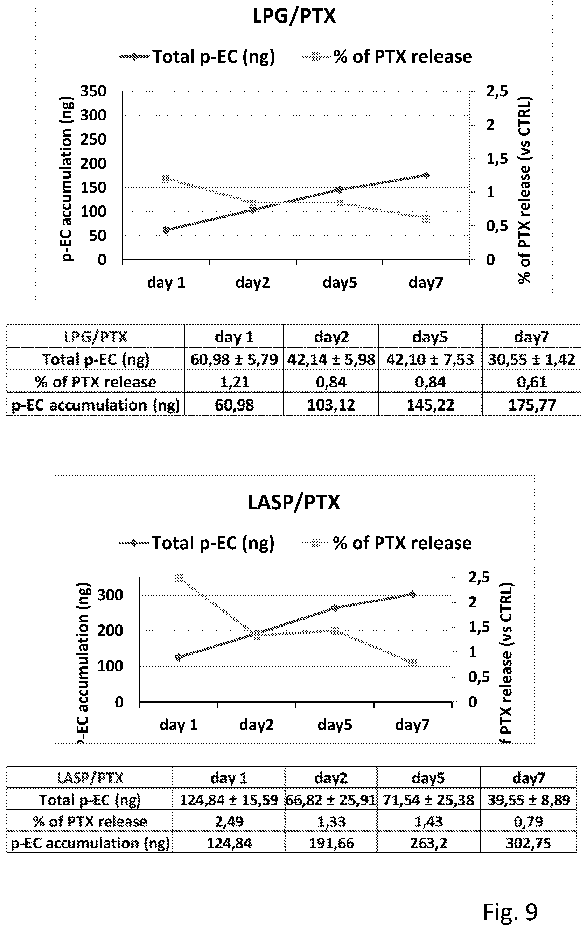

- Figure 9 shows the percentage of PTX releasing. The graphs and the tables show the release of PTX expressed as total amounts ( p-EC ) and percentage vs CTRL by both LPG/PTX (A) and LASP/PTX (B) at different days. P-EC stands for PTX equivalent concentration evaluated in a biological dosage assay.

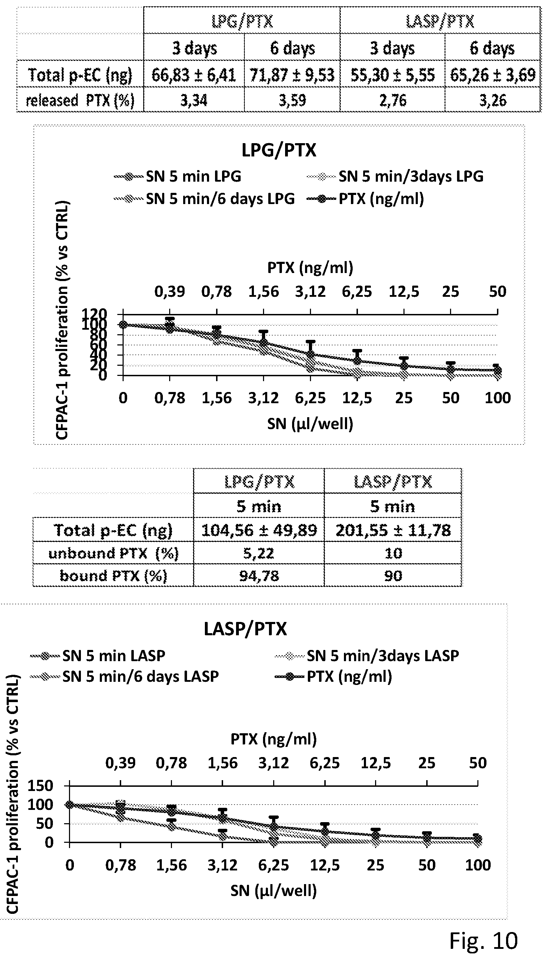

- Figure 10 shows the biological dosage of the bound/unbound PTX. The amount of bound/unbound PTX at 5 minutes, three and six days after the treatment. The dose response kinetics used to calculate the values reported in the Tables A and B.

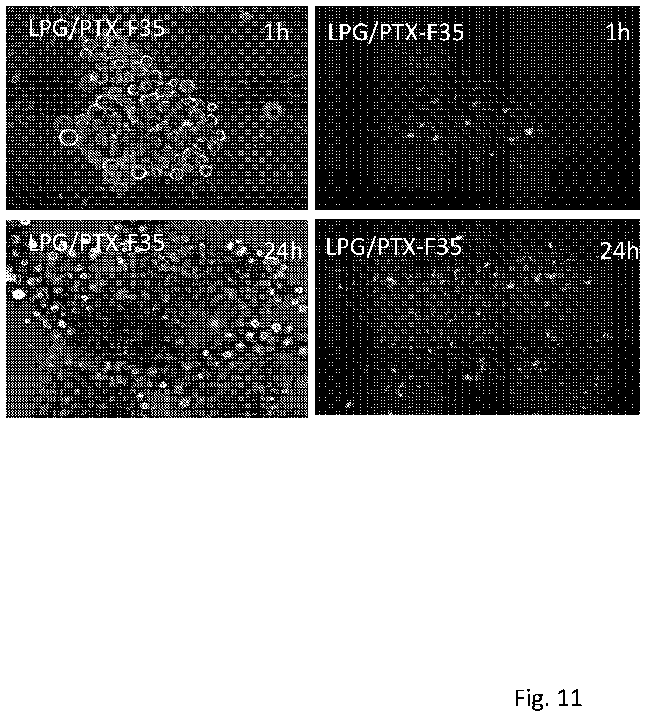

- Figure 11 shows the photos of LPG treated for 1h or 24h with Fluorescent PTX (PTX-F35) at 2ug/ml. The results show that 1h is sufficient for a complete uptake of PTX-F35 by LPG. The results show that PTX-F35 is mainly localized in the cytoplasm of LPG adipocytes.

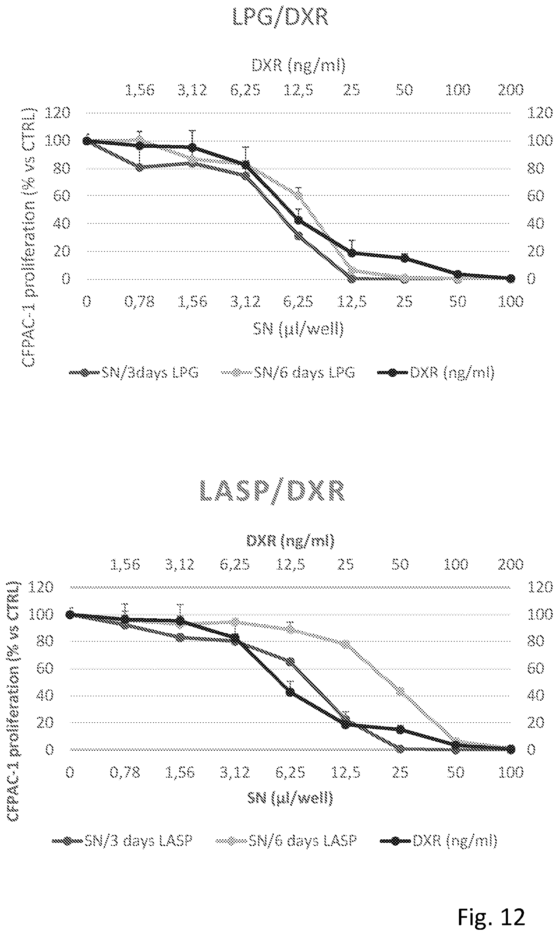

- Figure 12 shows the Doxorubicin (DXR) uptake and release by both LPG and LASP. The graphs report the biological activity of SN (surnatants) from both LPG (A) and LASP (B) that were treated with Doxorubicin (DXR).
   The SN were collected by washing the LPG/DXR and LASP/DXR after 3 and 6 days from the treatment and tested on CFPAC-1 cells.

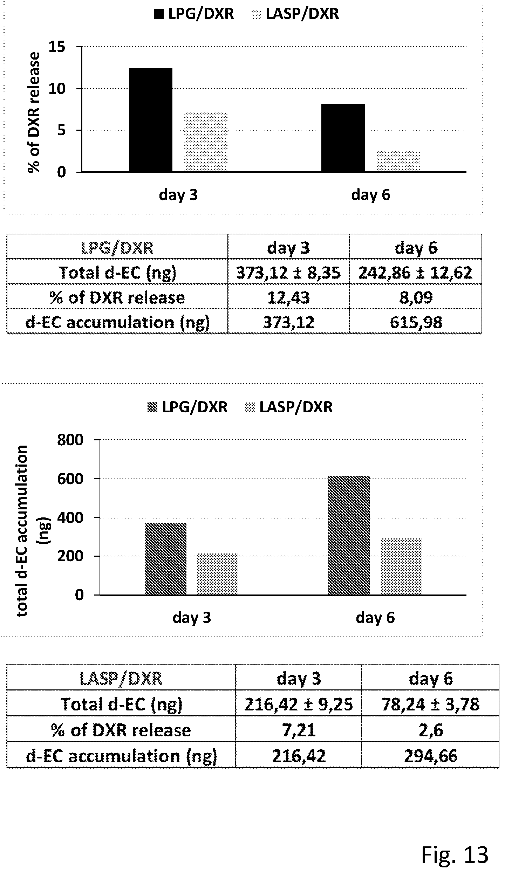

- Figure 13 shows the time releasing kinetics of DXR by both LPG/DXR and LASP/DXR. The histograms report the amount of DXR released by both LPG and LASP at days 3 and 6 after the treatment expressed as percentage of the amount used to treat LPG and LASP (A) and as total DXR equivalent concentration (d-EC) (B). Table (C) summarizes the numerical values.

### DETAILED DESCRIPTION OF THE INVENTION

A first object of the present invention refers to a scaffold loaded with drugs, wherein said scaffold is isolated fat tissue, wherein the isolated fat tissue is micro-fragmented fat or a micro-fragmented lipoaspirate and/or clusters of micro-fragmented fat/lipoaspirate comprising Mesenchymal Stem Cells (MSCs), Adipose-derived Stem Cells (ASCs), Adipose Stem Cells, pericytes, adipocytes and endothelial cells and wherein said drugs are selected from: antiinflammatory molecules, antibiotics, anti-cancer molecules, and 5a-Reductase inhibitors.

The tissue-based system allows the delivery of molecules and/or drugs in the body or any part of the body of an individual (any animal) in need thereof. Therefore, the tissue-based system of the invention allows molecules and/or drugs administration in individuals (any animal) in need thereof. Preferably, the molecules/drugs are delivered in the interested site, preferably the sick and/or the injured site.

In the contest of the present invention, fat tissue means adipose tissue. Preferably, said fat tissue is isolated from any animal, more preferably it is isolated from a humans said humans being alive or a cadaver.

Preferably, said fat tissue derives/is isolated (purified) from any part of the body, preferably from the lower and/or the lateral abdomen area. Preferably, said fat tissue is isolated from the body by lipoaspiration/liposuction (lipoaspirate) procedure. Therefore, according to a preferred embodiment the fat tissue is a lipoaspirate (LASP in the example and drawings as an example of fat tissue) or derivatives thereof. In the context of the present invention, lipoaspiration or liposuction or simply lipo means the removal of adipose tissue (fat) under negative pressure condition, generally by using a cannula.

As already mentioned before, the fat tissue, preferably the lipoaspirate, is micro-fragmented (LPG in the example and drawings as an example of micro-fragmented fat tissue). Preferably, the fat tissue is micro-fragmented by a non-enzymatic procedure and therefore the fat of the present invention is more preferably non-enzymatic micro-fragmented fat. In other words, the fat used/administered in the present invention as delivery system has been micro-fragmented without any enzymatic treatment.

According to a further preferred embodiment of the present invention, the micro-fragmented fat tissue is obtained by using the Lipogems^{®} device (LPG), more preferably according to the procedure as fully disclosed in the patent application WO2011/145075.

The fat tissue, preferably the lipoaspirate, is introduced in the Lipogems^{®} device wherein it is progressively reduced (fragmented) in small clusters of fat tissue preferably by means of mild mechanical forces and, more preferably, in presence of a solution, preferably a saline solution. According to a preferred embodiment, the micro-fragmented fat of the invention contains clusters of fat tissue having size ranging preferably from 10 to 5000 µm, more preferably from 100 to 3000 µm, still more preferably from 200 to 2500 µm, more preferably from 300 to 1500 µm, more preferably from 400 to 900 µm.

The micro-fragmented fat or the clusters of micro-fragmented fat, comprise Mesenchymal Stem Cells (MSCs) and /or Adipose-derived Stem Cells (ASCs) and/or Adipose Stem Cells and/or pericytes and/or adipocytes and/or endothelial cells. In this regard, particularly advantageous are the micro-fragmented fat clusters since they keep the natural/intact stromal vascular niche of the resident cells that, consequently, are supported by the stroma resembling the natural/physiological context in trophic and/or signaling terms. Additionally, the stroma provides a protected environment during the graft of the cells against any physical and/or chemical insults, such as mechanical, oxygen, ecc.

Therefore, the micro-fragmented fat of the present invention is preferably characterized by:
- Clusters of tissue having size ranging preferably from 10 to 5000 µm, more preferably from 100 to 3000 µm, still more preferably from 200 to 2500 µm, still more preferably from 300 to 1500 µm, still more preferably from 400 to 900 µm; and/or
- Free from blood residues and/or pro-inflammatory oily substances.

According to a preferred embodiment, the isolated fat tissue comprises cells expressing at least one, preferably all, marker selected from: CD44, CD73, CD90, CD105, CD146 CD166 and any combination thereof; and/or at least one marker, more preferably all, selected from: OCT4, SOX2, NANOG, b-tubulin III NESTIN, NEUROD1, MUSASHI1, PAX6, SOX3 and any combination thereof.

More preferably, the cells, preferably said Mesenchymal Stem Cells (MSCs) and/or Adipose-derived Stem Cells (ASCs) and/or Adipose Stem Cells co-express the following panel of markers (signature): nestin, b-tubulin III, GFAP, and O4.

Fat fragmentation inside the device is preferably controlled by using one or more fragmentation/disaggregation/emulsifying means.

According to a preferred embodiment, said means are metallic means, more preferably metallic beads and/or filters/nets, wherein the filters/nets provide preferably a micro-fragmentation of the tissue sample, while the beads freely move inside the device in order to promote the separation between the solid part and the liquid part of the tissue sample and (inherently) provide an emulsion of the liquid parts with the a washing fluid. Preferably the beads have size (average diameter) ranging preferably from 0,1-30 millimeters, more preferably 1-20 mm, still more preferably 5-10 mm, still more preferably 7,5-8,5 mm and/or said filter/nets have average diameter ranging from 2000 µm to 200 µm, preferably from 1500 µm to 500µm.

The mesh average diameter (pore size) of the filter/net ranges between 50µm and 6000µm, preferably between 500µm and 3000µm.

It is advisable to perform mild movements of the fragmentation/disaggregation/emulsifying means throughout the fat tissue, more preferably by performing a controlled shaking of the device.

According to a preferred embodiment, the fragmentation/disaggregation/emulsification is performed in immersion, preferably with a continuous flow of saline buffer through the device, so allowing an easy washing of the tissue sample (in particular an effective oil and/or blood residues removal). More preferably, the fragmentation/disaggregation/emulsification is performed by washing the tissue sample through a continuous flow of the saline buffer that, together with beads shaking, allows the solid material to lift towards the inlet of the saline buffer, leaving the oil and/or blood residues to flow together with the saline towards the outlet.

The fragmentation/disaggregation/emulsification procedure lasts for preferably few seconds.

Therefore, according to a further preferred embodiment, the micro-fragmented fat of the present invention is obtained by using a gentle, enzyme-free, sterile, intra-operative and rapid manipulation.

The fat tissue of the present invention is preferably isolated from any animal, more preferably from humans. Preferably said animal/human is healthy or cadaver.

According to a preferred embodiment, the fat is animal adipose tissue, more preferably human adipose tissue, more preferably isolated/lipoaspirate from the lower and/or the lateral abdomen area of an individual. However, said fat can be isolated from any useful body area.

Preferably, the micro-fragmented fat is autologous or heterologous.

For the purpose of the present invention, the molecules to be delivered are selected from: anti-inflammatory molecules, antibiotics, anti-cancer molecules, and 5α-Reductase inhibitors (5-ARIs). Said anti-cancer molecules (chemotherapeutics) are preferably selected from: natural products, preferably vinca alkaloids, more preferably selected from: vinblastine, vincristine, and vinorelbine, taxane, preferably paclitaxel or docetaxel, vincristin, vinblastin, nocodazole, epothilones and navelbine, epidipodophyllotoxins (teniposide), actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamineoxaliplatin, iphosphamide, melphalan, merchlorethamine, mitomycin, mitoxantrone, nitrosourea, plicamycin, procarbazine, teniposide, triethylenethiophosphoramide and etoposide (VP16)), adriamycin, amsacrine, camptothecin, daunorubicin, dactinomycin, doxorubicin, eniposide, epirubicin, etoposide, idarubicin, irinotecan (CPT-11) and mitoxantrone, pemetrexed, 5-FU, rafenib, metotrexate, cyclophosphamide, bortezomib, tomozolomide, sorafenib. Any combination of the molecules reported above should be considered forming part of the present disclosure.

More preferably, the molecules are selected from: Paclitaxel (PTX - Taxol or Onxal) or derivatives thereof, preferably selected from Abraxane and/or Docetaxel, doxorubicin or derivative thereof, preferably Adriamycin and/or, Vincristine and any combination thereof.

The anti-cancer molecules can be delivered also in combination with further molecules, preferably selected from: antibiotics, anti-inflammatory substances, poli- or mono-clonal antibodies, immunomodulatory molecules, biological drugs and combinations thereof.

The molecule and/or the drug/prodrug can be modified in any way, such as pegylation or it can be associated with particles, preferably nanoparticles, such as albumin-nanoparticles.

According to a further aspect of the present invention, the tissue-based delivery system of the invention, loaded/primed with anti-cancer molecules and/or drugs as disclosed above, is used for the treatment of a cancer.

In the context of the present invention the term "cancer" refers to any neoplastic disorder, including such cellular disorders as, for example, renal cell cancer, Kaposi's sarcoma, chronic leukemia, prostate cancer, breast cancer, sarcoma, pancreatic cancer, ovarian carcinoma, rectal cancer, throat cancer, melanoma, colon cancer, bladder cancer, mastocytoma, lung cancer, mammary adenocarcinoma, pancreatic adenocarcinoma, myeloma, lymphoma, pharyngeal squamous cell carcinoma, and gastrointestinal or stomach cancer.

The most preferred type of cancer to be treated by using the delivery system of the present invention is selected from: pancreatic cancer, glioblastoma, neuroblastoma, mesothelioma, ovarian carcinoma, and prostatic cancer, and mammary adenocarcinoma.

Alternatively, the delivery system of the invention, loaded/primed with at least one molecule and/or drug as disclosed above, is used for the treatment of any disease or condition associated with or caused by an altered and/or increased growth state, preferably a hyperproliferative disease/disorder.

The "growth state" of a cell refers to the rate of proliferation of the cell and/or the state of differentiation of the cell.

As used herein, "hyperproliferative disease/disorder" refers to any disorder, which is caused by or is manifested by unwanted proliferation of cells in a patient. Preferably, hyperproliferative disorders are selected from: psoriasis, rheumatoid arthritis, lamellar ichthyosis, epidermolytic hyperkeratosis, restenosis, endometriosis, and abnormal wound healing, or neuro degenerative diseases, preferably amyotrophic lateral sclerosis, spinal muscular atrophy, multiple sclerosis, and traumatic neural injury, such as spinal cord lesion.

As used herein, "proliferating" and "proliferation" refer to cells undergoing mitosis.

According to a preferred embodiment, the amount said molecules/drugs that can be loaded/primed into the delivering system of the invention ranges from 1 to 5 mg/ml of fat tissue.

According to a further preferred embodiment, the amount of Paclitaxel (PTX - Taxol or Onxal) or derivatives thereof, preferably Abraxane, Docetaxel, for obtaining an anti-cancer effect/activity is not less than 150 ng for 100ul of micro-fragmented fat tissue/lipoaspirate (LPG) and/or not less than 300 ng for 100ul of fat tissue/lipoaspirate(LASP).

Nevertheless, the amount that can be loaded as maximum depends on the lipo-hydrophylic nature of the drug.

The amount of the molecules/drugs released per day by the delivery system of the invention ranges from 10-15% compared to the loading/priming amount that is the amount used to prime the micro-fragmented fat tissue/lipoaspirate (LPG) and/or fat tissue/lipoaspirate (LASP).

A further aspect of the present invention refers to the delivery system of the invention, loaded with at least one molecule and/or drug as disclosed above for use in the treatment of a disease or a condition caused by or associated with impaired (altered) angiogenesis, therefore for treating pathological angiogenesis.

Besides cancer, in the context of the present invention, for disease/condition associate with or caused by altered angiogenesis is meant further diseases such as diabetic retinopathy or neuropathy.

According to a preferred embodiment of the invention, the tissue-based delivery system is for local, parenteral, peritoneal, mucosal, dermal, epidermal, subcutaneous, transdermal, intramuscular, nasal, oral, topical, vaginal, rectal or intra-ocular administration.

According to a further preferred embodiment, the tissue-based delivery system of the invention, loaded with the molecules/drugs as disclosed above, is administered/applied in combination (pre-post) radiotherapy and/or surgery. Preferably the tissue-based delivery system of the invention, loaded with the molecules/drugs as disclosed above, is applied on the interested area before surgery for example in order to reduce the tumor area to be removed and therefore, to make the surgery less traumatic especially for specific area such as brain/head.

The tissue-based delivery system of the invention, loaded with the molecules/drugs as disclosed above, is preferably pre and/or post-operatory administration/application, preferably topical, intraperitoneal, subcutaneous, administration/application, preferably for preventing the cancer relapses, more preferably for metastatic tumors.

### EXAMPLE

### Sample collection

The following results refer to both lipoaspirate as such (LASP, not according to the invention) and Lipogems^{®} tissue (LPG that is the lipoaspirate after treatment with Lipogems device - micro-fragmented lipoaspirate).

LASP has been obtained by liposuction of subcutaneous tissue as previously described (WO2011/145075) by using disposable cannulas provided with the Lipogems^{®} kit.

In order to obtain micro-fragmented adipose tissue - the LPG -, the LASP was processed by the Lipogems^{®} device according to Bianchi et al., 2013 and Tremolada et al., 2016.

### Drugs

LPG and LASP samples were exposed to the following example of chemotherapy drugs:
1) Paclitaxel (PTX - stock solution 6 mg/ml);
2) Doxorubicin hydrochloride, and
3) Vincristine (VC) (stock solution 5 mg/ml).

PTX, DXR and VC are diluted in culture medium as reported below at the working/requested concentration.

### Priming of Lipogems^{®} and Lipoaspirate with drug

Samples of both LPG and LASP were vortexed for 1 minute in 15 ml conical tube (Euroclone, UK). Subsequently, PTX, DXR and VC were added to the samples at a final concentration of 2 µg/ml.

The samples LPG and LASP were vortexed 1 minute and then incubated 5 minutes or 24 hours at 37°C, 5% CO2. After the incubation the samples were mixed with 1 volume of Iscove complete medium (IMDM +10% FBS + 2mM L-glutamine; Euroclone, UK), further vortexed 1 minute and centrifuged at 2500 xG, 10 min.

The hydrophilic phase was immediately collected and replaced.

This operation was repeated at different times as described in each experiment.

The PTX, DXR, VC-primed samples (LPG/PTX, LPG/DXR, LASP/PTX, LASP/DXR, LPG/VC, LASP/VC) were then processed according to different methodology to study the drug release.

### Cell lines

The anticancer activity of PTX, DXR and VC was tested on the following cells:
- a human pancreatic adenocarcinoma cell line CFPAC-1 cultured and amplified in Iscove complete medium;
- a primary Glioblastoma (GBM) cell line, named GC-GBM, cultured and amplified in Neurocult Medium (NC); and
- the following Neuroblastoma (NB) cell lines: IMR32, HTLA230, SY5Y, SY5Y-LUC, NB1691 and NB1691-Luc that were gently donated by Dr. Mirco Ponzoni, (Gaslini Hospital Genova, Italy).

SY5Y-Luc, NB1691 and NB1691-Luc cells were cultured in RPMI 1640 + 10%FCS.

IMR32, HTLA230 and SY5Y cells were cultured in DMEM complete medium ((Euroclone, UK) and passed every 72h at split ratio 1:5.

The anti-angiogenic activity of the LPG and LASP loaded with drugs has been assayed on human endothelial cell line (HUVEC).

HUVECs were grown in EGM completed medium (Lonza) and passed weekly 1:3

### Anticancer activity of LPG/PTX and LASP/ PTX analyzed by transwell inserts

Increasing volumes (25, 50, 100, 200 µl) of LPG/PTX and LASP/PTX were introduced in 24-well plate (BD Falcon, USA, diameter 1.9 cm2) using complete IMDM medium to a final volume of 700 µl. 2*103 CFPAC-1 cells in 300 µl of medium have been seeded into the upper insert (0.4 µm pore size; BD Falcon, USA).

The effect of the drugs released by the LPG/LASP on the growth of the tumor cells has been evaluated by staining the adherent cells with 0.25% crystal violet (Sigma Aldrich, USA) for 10 min followed by cell lysing with 0.5 mL of 33% glacial acetic acid.

The optical density (OD) of the eluted dye was measured at 550 nm (ChroMate, Awareness technology Inc, USA).

The medium of the inserts was collected to test the anticancer activity in a standardized biological dosage procedure to estimate the Paclitaxel equivalent concentration (p-EC) according to a MTT assay.

In some experiments, the cells were detached and counted to evaluate their number.

The results are expressed as percentage of growth inhibition referred to control cells growing in the absence of treatment (CTRL).

### Biological dosage of anticancer activity by MTT assay

The supernatants (SN) from LPG and LASP primed with drugs were evaluated by MTT assay (3-(4,5-dimethyl-2-thiazolyl)-2,5-diphenyl-2-H-tetrazolium; Sigma-Aldrich, USA) on CFPAC-1 cell proliferation (Mosmann, 1983).

The inhibitory concentration (IC50) was determined according to the Reed and Muench formula (1938).

According to a biological dosage system, the anti-tumour activity of PTX and DXR primed LPG and LASP was compared to that of free drugs and expressed as PTX or DXR-equivalent concentration (respectively p-EC and d-EC), applying the following algorithm: p- or d-EC(ng/ml) = IC50 PTX or DXR (ng/ml)×100/ V50-SN; where the V50-SN is the volume of SN from primed LPG or LASP at which the 50% of inhibition was observed and IC50 PTX or DXR is the concentration (ng/ml) of pure PTX or DXR producing 50% of inhibition.

In order to evaluate the percentage of PTX or DXR released by the primed LPG or LASP (drug release %) we calculated the total amount of drug released (p-EC or d-EC x volume of SN) referred to the total amount (µg) of drug used to prime LPG or LASP.

### Time releasing kinetics of drug by LPG/PTX and LASP/PTX

LPG/PTX and LASP/PTX were diluted into equal volume of Iscove complete medium (Euroclone, UK). The samples were vortexed for one minute and then incubated at 37°C, 5%CO2 and at different times of incubation (1, 2, 3, 5 and 7 days). The medium supernatant (SN) was collected to test its anticancer activity in vitro. Supernatants from unprimed LPG or LASP were used as control.

### Data elaboration

The results are expressed as mean ± standard deviation (SD). Where requested, the differences between mean values were evaluated according to Student's t-test performed by GraphPad InStat program (GraphPad Software Inc., San Diego, CA, USA). p-Values ≤ 0.05 were considered statistically significant. The dose-response kinetics were analysed by using linear regression and evaluating the correlation coefficient (R2) by Excel 2007 software (Microsoft, Inc.).

### RESULTS

### LPG/PTX and LASP/PTX in vitro inhibition of the cancer growth

The results show that increasing amount (25, 50, 100 and 200 µl) of primed LPG (LPG/PTX) or LASP (LASP/PTX) produced a dramatic and complete inhibition of the growth of CFPAC-1 (Fig 1 A).

The observed inhibition is maximal also at the lowest amount of sample (Fig.1 B) meaning that the amount of PTX released in 1 ml of medium by 25 µl of the primed sample reached a concentration of IC90. Therefore, in order to estimate the PTX equivalent concentration (p-EC) in the transwell, we performed a biological dosage of the culture medium receiving 25, 50, 100 and 200 µl of LPG/PTX or LASP/PTX that was mixed to a final volume of 1 ml of medium (corresponding to a dilution of 1:40; 1:20; 1:10 and 1:5). The results show that the media (hydrophilic) produced a dose response inhibition with very high anticancer activity as indicated by the V50 value (volume inhibiting 50% cell growth) reported in the box (Fig.2).

The calculation of total PEC values (Fig.3) confirmed that the releasing of PTX is dependent on the amount of primed samples of LPG or LASP. Indeed, the results show that there is a significant correlation well expresses by the R2 values that resulted of 0.86 and 0.75 for LPG/PTX and LASP/PTX respectively.

Similar results were obtained by testing LPG/PTX and LASP/PTX on CG-GBM cell line and on NB cell line IMR32. In particular, the results on these cells show that the addition of SN at different dilutions derived from LPG/PTX and LASP/PTX to culture of GC-GBM and IMR32 cells produced a potent dose dependent growth inhibition. Additionally, GC-GBM and IMR32 cells were treated with LPG/PTX or LASP/PTX derived SN, obtained after different time of incubation. At each time of incubation, SN was aspirated and replaced with fresh medium. These experiments were performed to assess the antitumor effect duration. The results show that the antitumor effect of LPG/PTX on GC-GBM and IMR32 respectively was maintained even after 4 weeks of incubation.

In particular, the activity of LASP/PTX decreased more rapidly.

Further, we performed experiments with PTX to establish the dose of drug sufficient to load 100 µl of LPG or LASP.

The results show that the minimal dose necessary of PTX to obtain an efficient antitumor effect was around 150 ng for 100ul of LPG and 300ng for 100ul of LASP on both tumor cell lines.

The morphological appearance of GC-GBM and IMR32 cancer cells upon 72h treatment with LPG/PTX and LASP/PTX derived SN and control fat SN is shown in Fig. 4 and 5.

The capacity of LPG and LASP loaded with PTX, DXR and VC of inhibiting cancer cell proliferation was confirmed by using other NB tumor cell lines.

Anti-angiogenic activity of LPG and LASP loaded with anticancer drugs

In order to evaluate the capacity of LPG/PTX and LASP/PTX to affect angiogenesis HUVECs were used. The experiments disclosed above referring to the cancer cell lines were repeated using HUVECs (Fig.6).

The results show that SNs derived from LPG/PTX and LASP/PTX are able to inhibit in a dose dependent manner the growth of HUVECs. On the other end, SNs from control untreated LPG and LASP did not affect the growth of HUVECs (Fig.6A). The results show that the anti-angiogenic effect of LPG/PTX is really high and as observed for the cancer cells, it was maintained effective for up to 4 weeks.

In particular, LASP/PTX show a reduced effect compared to the one of LPG/PTX (Fig.6B).

Interestingly, the amount of PTX needed to induce anti-angiogenic effects is less than 150ng for 100ul of LPG; instead, in order to have similar antiangiogenic activity with LASP, 600 ng of PTX was required (Fig.6C). The morphological analysis of HUVECs upon exposure to LPG/PTX or LASP/PTX show a potent nuclear fragmentation of the cells suggesting that the mechanism of LPG/PTX or LASP/PTX on HUVECs growth inhibition is probably associated with cell apoptosis.

Similar results were seen by using VC instead of PTX.

Thawing and freezing did not affect LPG capacity to load and release drugs.

Indeed, samples of LPG were kept at -20°C for 1 week and then thawed and treated with PTX. Alternately, fresh LPG samples were treated with PTX and frozen, maintained at -20°C for 1 week and then thawed to test antitumor and anti-angiogenic activity.

LPG samples, frozen either before or after the treatment with PTX, keep their antitumor and anti-angiogenic activities (Fig.7).

The same results were observed when LPG was loaded with DXR and VC or when LASP was used.

However, in this case, a significant decrease of the antitumor efficacy was observed if drug was loaded upon LASP thawing.

Time releasing kinetics of drug by LPG/PTX and LASP/PTX

In order to evaluate the amounts of PTX released by the primed LPG and LASP a standard macro-system in tubes has been used.

The study was setup by priming 2 ml of sample with 2000 ng/ml of PTX for 5 hours and then directly mixing it with the same volume (1:2) of culture medium.

This allowed verifying the efficacy of a short time of priming (5hs) and the subsequent release until one week. The sample was incubated at 37° C and at day 1, 3, 5 and 7 after sample centrifugation, the medium (hydrophilic fraction) was collected and replaced with new medium. The media have been collected at the different days for evaluating their biological activity on cancer cells. The results demonstrate high activity until the last days of culture tested (Fig.8).

### Estimation of percentage of releasing by LPG/PTX and LASP/PTX

The biological assay of PTX anticancer activity, allowed estimating the amount of drug released in the medium per day in term of p-EC. The release is referred to the amount of PTX used to prime LPG or LASP (that is 5.000 ng) and is expressed as the percentage of drug released and as a kinetics of drug accumulation (Fig.9).

The results show that the total amount of PTX released at day 1 is 60.9 ng for LPG/PTX and 124.8 ng for LASP/PTX.

The medium has been replaced at each time. Therefore, we can estimate a kinetic of drug accumulation by integrating the amount of PTX released at each time. A day 7 it is of 175.7 ng for LPG/PTX and 302.7 ng for LASP/PTX.

Moreover, the results show a decrease of the percentage of the released PTX along the time.

In view of this observation, the possible correlation between the percentage of drug release and the dilution of the sample has been evaluated in order to better appreciate the drug releasing kinetics.

The results show a significant correlation between the percentage of drug release related and the increasing of the sample dilution (this is more evident for LPG/PTX, R2 =0.91 than LASP/PTX R2=0.52). This observation could be explained by the lipophilic chemical structure of PTX that can be easier eluted from LPG or LASP (in which is strongly incorporated) at higher dilutions of the hydrophilic medium. The higher is the dilution the higher is the amount of serum albumin that is a molecule having a significant PTX affinity.

These data demonstrate that LPG/LASP is able to release all along the tested days a pharmacological effective amount of the drug, the PTX in this case, in particular for the tested drug, an effective anticancer activity. Therefore, LPG and/or LASP can be used to release an effective drug amount (very high dosage) in the tumour area for several days, in particular, by the in situ injection.

Moreover, we can also argue that the efficiency of release in vivo can be facilitate by the biological structure of the surrounding tissue into which LPG/PTX or LASP/PTX are injected (e.g. if the environment is more or less lipophilic). In any case, LPG or LASP used to release drug in the tumour environment could reduce the systemic toxicity that occurs by intravenously chemotherapy.

### Study of PTX binding capacity to whole LPG and LASP

Experiments have been set up to measure the time the PTX needs to bind LPG or LASP and the rate of bound/unbound drug. In particular, the priming of the samples has been performed at different time (72, 24, 5 hours and 5 minutes).

The results are the same at any time therefore, here has been reported only the binding of PTX at 5 minutes.

After priming, the samples were immediately washed with the medium and centrifuged to collect the unbound fraction of PTX.

The floating fraction was also cultured in the medium for measuring the drug release after 3 and 6 days.

The results confirm that LPG bound about 95% of PTX (and 90% for LASP). Furthermore, after three and six days, PTX is still released in an amount able to exert a significant inhibition of the tumour cell proliferation (Fig.10).

The distribution of the drug in LPG has been evaluated by using a fluorescent PTX (PTX-F35). The results show that PTX-F35 (2ug) is mostly adsorbed in the lipid fraction of LPG in one hour (Fig.11) and no free drug has been detected. The results are the same until 24hours. LASP shows the same drug distribution even if it appears less uniform.

### Preliminary doxorubicin experiments

In order to understand if LPG and LASP represents a good scaffold (delivery system) for non-lipophilic drugs the same experiments have been performed by using the hydrophilic antineoplastic drug Doxorubicin (DXR). The biological dosage of DXR released in the medium after three and six days its replacement clearly show that both LPG and LASP are able to bind and then release DXR in amount effective on in vitro tumor growth. Although to be confirmed by further data LPG seems to work better in term of releasing being at day 6 almost two times higher than that released at day 3 (Fig.12 and 13).

## Claims

1. A scaffold loaded with drugs, wherein said scaffold is isolated fat tissue, wherein the isolated fat tissue is micro-fragmented fat or a micro-fragmented lipoaspirate and/or clusters of micro-fragmented fat/lipoaspirate comprising Mesenchymal Stem Cells (MSCs), Adipose-derived Stem Cells (ASCs), Adipose Stem Cells, pericytes, adipocytes and endothelial cells and wherein said drugs are selected from: anti-inflammatory molecules, antibiotics, anti-cancer molecules, and 5α-Reductase inhibitors.

2. The scaffold according to claim 1 wherein the isolated fat tissue comprises cells expressing CD44, CD73, CD90, CD105, CD146 and CD166.

3. The scaffold according to claim 2 wherein the cells express OCT4, SOX2, NANOG, b-tubulin III, nestin, NEUROD1, MUSASHI1, PAX6 and SOX3.

4. The scaffold according to claim 2 or 3, wherein the cells express nestin, b-tubulin III, GFAP and O4.

5. The scaffold according to any one of claims 1 -4, wherein the fat tissue, preferably the micro-fragmented fat tissue or the micro-fragmented lipoaspirate, is isolated from any animal, more preferably it is isolated from humans said humans being alive or cadaver.

6. The scaffold according to any one of claims 1-5 wherein said anti-cancer molecules (chemotherapeutics) are selected from: vinca alkaloids, preferably selected from: vinblastine, vincristine, and vinorelbine, taxane, preferably paclitaxel or docetaxel, vincristin, vinblastin, nocodazole, epothilones and navelbine, epidipodophyllotoxins (teniposide), actinomycin, amsacrine, anthracyclines, bleomycin, busulfan, camptothecin, carboplatin, chlorambucil, cisplatin, cyclophosphamide, Cytoxan, dactinomycin, daunorubicin, doxorubicin, epirubicin, hexamethylmelamineoxaliplatin, iphosphamide, melphalan, merchlorethamine, mitomycin, mitoxantrone, nitrosourea, plicamycin, procarbazine, teniposide, triethylenethiophosphoramide and etoposide (VP16)), adriamycin, amsacrine, camptothecin, daunorubicin, dactinomycin, doxorubicin, eniposide, epirubicin, etoposide, idarubicin, irinotecan (CPT-11) and mitoxantrone, pemetrexed,5-FU, rafenib, metotrexate, cyclophosphamide, bortezomib, tomozolomide, sorafenib, and any combination thereof.

7. The scaffold according to claim 6, wherein said anti-cancer molecules are selected from: Paclitaxel (PTX - Taxol or Onxal), Abraxane and/or Docetaxel, doxorubicin, Adriamycin or Vincristine and any combination thereof.

8. The scaffold according to claim 6 or 7, wherein said anticancer molecules are selected from: Paclitaxel (PTX - Taxol or Onxal), doxorubicin, Vincristine and any combination thereof.

9. The scaffold according to any one of claims 1-8, wherein the amount of said drugs ranges from 1 to 5 mg/ml.

10. The scaffold according to claim 9 wherein the amount of Paclitaxel (PTX - Taxol or Onxal) Abraxane, Docetaxel is not less than 150 ng for 100µl of said micro-fragmented fat tissue or micro-fragmented lipoaspirate or not less than 300 ng for 100µl of fat tissue or lipoaspirate.

11. The scaffold loaded with drugs according to any one of claims 1-5 wherein said drugs are anticancer molecules according to any one of claims 6 -10, for use in the treatment of a cancer.

12. The scaffold for use according to claim 11 wherein said cancer is selected from: renal cell cancer, Kaposi's sarcoma, chronic leukemia, prostate cancer, breast cancer, sarcoma, pancreatic cancer, ovarian carcinoma, rectal cancer, throat cancer, melanoma, colon cancer, bladder cancer, mastocytoma, lung cancer, mammary adenocarcinoma, pancreatic adenocarcinoma, myeloma, lymphoma, pharyngeal squamous cell carcinoma, and gastrointestinal or stomach cancer.

13. The scaffold for use according to claim 12 wherein said cancer is selected from: pancreatic cancer, glioblastoma, neuroblastoma and mesotelioma.

## Patentansprüche

1. Ein mit Wirkstoffen beladenes Gerüst, wobei das Gerüst isoliertes Fettgewebe ist, wobei das isolierte Fettgewebe mikrofragmentiertes Fett oder ein mikrofragmentiertes Lipoaspirat und/oder Cluster aus mikrofragmentiertem Fett/Lipoaspirat ist, die mesenchymale Stammzellen (MSCs), aus Fettgewebe gewonnene Stammzellen (ASCs), Fettgewebe-Stammzellen, Perizyten, Adipozyten und Endothelzellen umfassen, und wobei die Wirkstoffe ausgewählt sind aus: entzündungshemmenden Molekülen, Antibiotika, Antikrebsmolekülen und 5α-Reduktase-Inhibitoren.

2. Gerüst nach Anspruch 1, wobei das isolierte Fettgewebe Zellen umfasst, die CD44, CD73, CD90, CD105, CD146 und CD166 exprimieren.

3. Gerüst nach Anspruch 2, wobei die Zellen OCT4, SOX2, NANOG, b-Tubulin III, Nestin, NEUROD1, MUSASHI1, PAX6 und SOX3 exprimieren.

4. Gerüst nach Anspruch 2 oder 3, wobei die Zellen Nestin, β-Tubulin III, GFAP und O4 exprimieren.

5. Gerüst nach einem der Ansprüche 1-4, wobei das Fettgewebe, vorzugsweise das mikrofragmentierte Fettgewebe oder das mikrofragmentierte Lipoaspirat, von einem beliebigen Tier isoliert wurde, besonders bevorzugt wurde es von lebenden oder toten Menschen isoliert.

6. Gerüst nach einem der Ansprüche 1-5, wobei die Antikrebsmoleküle (Chemotherapeutika) ausgewählt sind aus: Vinca-Alkaloiden, vorzugsweise ausgewählt aus: Vinblastin, Vincristin und Vinorelbin, Taxan, vorzugsweise Paclitaxel oder Docetaxel, Vincristin, Vinblastin, Nocodazol, Epothilonen und Navelbin, Epidipodophyllotoxinen (Teniposid), Actinomycin, Amsacrin, Anthracyclinen, Bleomycin, Busulfan, Camptothecin, Carboplatin, Chlorambucil, Cisplatin, Cyclophosphamid, Cytoxan, Dactinomycin, Daunorubicin, Doxorubicin, Epirubicin, Hexamethylmelaminoxaliplatin, Iphosphamid, Melphalan, Merchlorethamin, Mitomycin, Mitoxantron, Nitrosharnstoff, Plicamycin, Procarbazin, Teniposid, Triethylenthiophosphoramid und Etoposid (VP16)), Adriamycin, Amsacrin, Camptothecin, Daunorubicin, Dactinomycin, Doxorubicin, Eniposid, Epirubicin, Etoposid, Idarubicin, Irinotecan (CPT-11) und Mitoxantron, Pemetrexed, 5-FU, Rafenib, Metotrexat, Cyclophosphamid, Bortezomib, Tomozolomid, Sorafenib und einer beliebigen Kombination davon.

7. Gerüst nach Anspruch 6, wobei die Antikrebsmoleküle ausgewählt sind aus: Paclitaxel (PTX - Taxol oder Onxal), Abraxane und/oder Docetaxel, Doxorubicin, Adriamycin oder Vincristin und einer beliebigen Kombination davon.

8. Gerüst nach Anspruch 6 oder 7, wobei die Antikrebsmoleküle ausgewählt sind aus: Paclitaxel (PTX - Taxol oder Onxal), Doxorubicin, Vincristin und einer beliebigen Kombination davon.

9. Gerüst nach einem der Ansprüche 1-8, wobei die Menge der Wirkstoffe zwischen 1 und 5 mg/ml liegt.

10. Gerüst nach Anspruch 9, wobei die Menge an Paclitaxel (PTX - Taxol oder Onxal), Abraxane, Docetaxel nicht weniger als 150 ng pro 100 µl des mikrofragmentierten Fettgewebes oder mikrofragmentierten Lipoaspirats oder nicht weniger als 300 ng pro 100 µl Fettgewebe oder Lipoaspirat beträgt.

11. Das mit Arzneimitteln beladene Gerüst nach einem der Ansprüche 1-5, wobei es sich bei den Arzneimitteln um Antikrebsmoleküle gemäß einem der Ansprüche 6-10 handelt, zur Verwendung bei der Behandlung von Krebs handelt.

12. Gerüst zur Verwendung nach Anspruch 11, wobei die Krebsart ausgewählt ist aus: Nierenzellkrebs, Kaposi-Sarkom, chronischer Leukämie, Prostatakrebs, Brustkrebs, Sarkom, Bauchspeicheldrüsenkrebs, Eierstockkrebs, Rektumkrebs, Kehlkopfkrebs, Melanom, Dickdarmkrebs, Blasenkrebs, Mastozytom, Lungenkrebs, Mammakarzinom, Pankreasadenokarzinom, Myelom, Lymphom, Rachen-Plattenepithelkarzinom und Magen-Darm-Krebs oder Magenkrebs.

13. Gerüst zur Verwendung nach Anspruch 12, wobei die Krebsart ausgewählt ist aus: Bauchspeicheldrüsenkrebs, Glioblastom, Neuroblastom und Mesoteliom.

## Revendications

1. Echafaudage chargé avec des médicaments, dans lequel ledit échafaudage est du tissu gras isolé, le tissu gras isolé étant de la graisse micro-fragmentée ou un lipoaspirat micro-fragmenté et/ou des groupes de graisse/lipoaspirat micro-fragmenté comprenant des cellules souches mésenchymales (MSC), des cellules souches dérivées de l'adipose (ASC), des cellule souches d'adipose, des péricytes, des adipocytes et des cellules endothéliales et lesdits médicaments étant choisi parmi les molécules anti-inflammatoires, les antibiotiques, les molécules anti-cancéreuses et les inhibiteurs de 5α -réductase.

2. Echafaudage selon la revendication 1 dans lequel le tissu gras isolé comprend des cellules exprimant CD44, CD73, CD90, CD105, CD146 et CD166.

3. Echafaudage selon la revendication 2 dans lequel les cellules expriment OCT4, SOX2, NANOG, la b-tubuline III, la nestine, NEUROD1, MUSASHI1, PAX6 et SOX3.

4. Echafaudage selon la revendication 2 ou 3, dans lequel les cellules expriment la nestine, la b-tubuline III, GFAP et O4.

5. Echafaudage selon l'une quelconque des revendications 1 à 4, dans lequel le tissu gras, de préférence le tissu gras micro-fragmenté ou le lipoaspirat micro-fragmenté, est isolé d'un quelconque animal, davantage de préférence il est isolé d'êtres humains lesdits êtres humains étant vivants ou des cadavres.

6. Echafaudage selon l'une quelconque des revendications 1 à 5 dans lequel lesdites molécules anti-cancéreuses (chimiothérapeutique) sont choisies parmi les alcaloïdes vinca, de préférence choisies parmi : la vinblastine, la vincristine et la vinorelbine, le taxane, de préférence le paclitaxel ou le docétaxel, la vincristine, la vinblastine, le nocodazole, les épothilones et la navelbine, les épidipodophyllotoxines (téniposide), l'actinomycine, l'amsacrine, les anthracyclines, la bléomycine, le busulfan, la campthotécine, le carboplatine, le chlorambucil, le cisplatine, le cyclophosphamide, le cytoxan, la dactinomycine, la daunorubicine, la doxorubicine, l'épirubicine, l'hexaméthylmélamineoxaliplatine, l'iphosphamide, le melphalan, la merchloréthamine, la mitomycine, la mitoxantrone, la nitrosourée, la plicamycine, la procarbazine, le téniposide, le triéthylènethiophosphoramide et l'étoposide (VP16)), l'adriamycine, l'amsacrine, la campthotécine, la daunorubicine, la dactinomycine, la doxorubicine, l'éniposide, l'épirubicine, l'étoposide, l'idarubicine, l'irinotécan (CPT-11) et la mitoxantrone, le pemetrexed,5-FU, le rafenib, le métotrexate, le cyclophosphamide, le bortezomib, le tomozolomide, le sorafénib et l'une quelconque de leurs combinaisons.

7. Echafaudage selon la revendication 6, dans lequel lesdites molécules anti-cancéreuses sont choisies parmi le paclitaxel (PTX - taxol ou onxal), l'abraxane et/ou le docétaxel, la doxorubicine, l'adriamycine ou la vincristine et l'une quelconque de leurs combinaisons.

8. Echafaudage selon la revendication 6 ou 7, dans lequel lesdites molécules anti-cancéreuses sont choisies parmi le paclitaxel (PTX - taxol ou onxal), la doxorubicine, la vincristine et l'une quelconque de leurs revendications.

9. Echafaudage selon l'une quelconque des revendications 1 à 8, dans lequel la quantité desdits médicaments se situe dans la plage allant de 1 à 5 mg/ml.

10. Echafaudage selon la revendication 9 dans lequel la quantité d epaclitaxel (PTX - taxol ou onxal), d'abraxane, de docétaxel est de pas moins de 150 ng pour 100 µl dudit tissu gras micro-fragmenté ou du lipoaspirat micro-fragmenté ou de pas moins de 300 ng pour 100 µl de tissu gras ou de lipoaspirat.

11. Echafaudage chargé avec des médicaments selon l'une quelconque des revendications 1 à 5 dans lequel lesdits médicaments sont des molécules anti-cancéreuses selon l'une quelconque des revendications 6 à 10, destinées à être utilisées dans le traitement d'un cancer.

12. Echafaudage destiné à être utilisé selon la revendication 11 dans lequel ledit cancer est choisi parmi : le cancer de cellule rénale, le sarcome de Kaposi, la leucémie chronique, le cancer de la prostate, le cancer du sein, le sarcome, le cancer pancréatique, le carcinome des ovaires, le cancer rectal, le cancer de la gorge, le mélanome, le cancer du côlon, le cancer de la vessie, le mastocytome, le cancer du poumon, l'adénocarcinome mammaire, l'adénocarcinome pancréatique, le myélome, le lymphome, le carcinome de cellule squameuse pharyngienne et le cancer gastrointesinal ou de l'estomac.

13. Echafaudage destiné à être utilisé selon la revendication 12 dans lequel ledit cancer ledit cancer est choisi parmi : le cancer pancréatique, le glioblastome, le neuroblastome et le mésotéliome.
